# EUROPEAN PATENT SPECIFICATION

(11) **EP 2 609 063 B1**
(45) Date of publication and mention of the grant of the patent: **16.05.2018**
(21) Application number: 11752170.8
(22) Date of filing: 24.08.2011
(51) Int. Cl.: C07C 29/42, C07C 29/17, C07C 45/62

(54) **PROCESS FOR THE MANUFACTURE OF 3,7-DIMETHYL-1-OCTEN-3-OL**
VERFAHREN ZUR HERSTELLUNG VON 3,7-DIMETHYL-1-OCTEN-3-OL
PROCÉDÉ DE FABRICATION DE 3,7-DIMÉTHYL-1-OCTÈNE-3-OL

(30) Priority: 24.08.2010 CH 13612010; 24.08.2010 US 376390 P
(43) Date of publication of application: 03.07.2013
(73) Proprietor: DSM IP Assets B.V., 6411 TE Heerlen (NL)
(72) Inventor: BONRATH, Werner, CH-4303 Kaiseraugst (CH); TSCHUMI, Johannes, CH-4303 Kaiseraugst (CH); MEDLOCK, Jonathan, CH-4303 Kaiseraugst (CH)
(74) Representative: Steck, Melanie
(86) International application number: PCT/EP2011/064531
(87) International publication number: WO 2012/025559

(56) References cited:
- EP-A1- 0 816 321
- EP-A2- 0 034 804
- WO-A2-2008/086847
- OFNER A ET AL: "SYNTHETISCHES NEROLIDOL UND VERWANDTE C15-ALKOHOLE", HELVETICA CHIMICA ACTA, VERLAG HELVETICA CHIMICA ACTA, BASEL, CH, vol. 17, no. 7, 1 January 1959 (1959-01-01), pages 2577-2584, XP002043079, ISSN: 0018-019X, DOI: 10.1002/HLCA.19590420729
- M MORENO-MANAS ET AL: "METAL COMPLEXES IN ORGANIC SYNTHESIS-V ALKYLATIONS OF PENTANE-2,4-DIONE WITH ALLYLIC ALCOHOLS UNDER PALLADIUM CATALYSIS", TETRAHEDRON, vol. 37, no. 17, 1 January 1981 (1981-01-01), pages 3009-3015, XP55026591,
- GAUTIER J.A.: "N° 383 Réactivité des alcools acétyléniques. VI. Alcools acétyléniques dérivés de la diéthylamino-1 butanone-3", BULLETIN DE LA SOCIETE CHIMIQUE DE FRANCE, 1 January 1972 (1972-01-01), XP009195482,

## Description

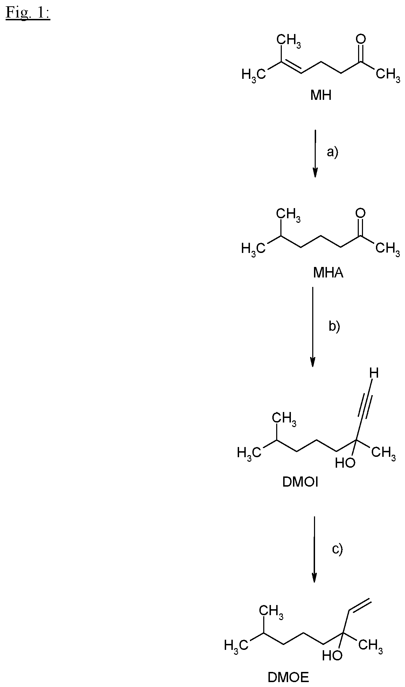

According to the process of the present invention (see Fig. 1) 6-methyl-5-hepten-2-on (MH) is hydrogenated to 6-methyl-2-heptanone (MHA) (step a), which is then reacted with acetylene to 3,7-dimethyl-1-octin-3-ol (DMOI) (step b). DMOI is then hydrogenated to 3,7-dimethyl-1-octen-3-ol (DMOE, step c).

MH itself can be prepared by reaction of isopropenyl methyl ether with 2-methyl-3-buten-2-ol as described by G. Saucy and R. Marbet in Helv. Chim. Acta 1967, 50, 2091-2095.

DMOE as well as tetrahydrolinalool (THLL, 3,7-dimethyl-octan-3-ol) are base products for the flavour and fragrance industry. DMOE, a colourless to pale yellow liquid, has a rose odour and is used in a variety of perfumery applications. It is seen as an alternative to geraniol.

DMOE and THLL are furthermore possible intermediates for isophytol (see e.g. P. Karrer, K.S. Yap, Helv. Chim. Acta 1940, 23, 581), which may be used to manufacture vitamin E (see Ullmann's Encyclopedia of Industrial Chemistry, Editors: Barbara Elvers, Stephen Hawkins, 5th completely revised edition, VCH Verlagsgesellschaft mbH, D-69451 Weinheim, 1996, Volume A27 (Thor to Vita), Chapter 4.11.2 - 4.11.5, page 484-488; especially Chapter 4.11.2 (page 484-485)).

6,10-dimethyl-5-undecen-2-one may be manufactured starting from DMOE according to processes known to the person skilled in the art like e.g. described in US 2,783,257 (see scheme in column 1) and DE-AS 1 193 490 (see example 10). 6,10-Dimethyl-5-undecen-2-one may then be hydrogenated to obtain hexahydropseudoionone (HPI) (see US 2,783,257: column 2, line 58-64), which may be further reacted to (iso)phytol according to processes as e.g. disclosed in Appl. Catal. 2005, 280, 55-73 and Catal. Today 2007, 121,45-57.

Processes for the manufacture of THLL and DMOE are already known:
P. Karrer and K.S. Yap (Helv. Chim. Acta 1940, 23, 581-584.) describe a procedure for the synthesis of THLL starting from MH. The MH reduction was carried out in the presence of a Pt catalyst, yields were not given. The resulting methylheptanone (MHA) was ethynylated using ethyne and sodium amide. 3,7-Dimethyl-octin-1-3-ol was catalytically reduced in presence of a Pt catalyst. The disadvantage of the procedure is that stoichiometric amounts of the ethynylation agent are used, the hydrogenations are performed with an expensive catalyst, no yields are given (the analysis is not discussed), the reactions are carried out in solvents, and low yields are obtained if we assume that 20 g of MH give 8 g of THLL, based on the experimental procedures reported. The THLL obtained is used for the synthesis of isophytol.

According to US 2,780,658 DMOE (named as 1,5-dimethyl-1-vinyl-1-hexanol) is synthesized starting from MHA which is ethynylated in presence of sodium in liquid ammonia followed by Lindlar hydrogenation in the presence of a catalyst which is Pd/Pb on CaCO₃. The hydrogenation was carried out in petroleum ether at 20-30°C. For the hydrogenation of 308 g of the starting material 20 g of the catalyst (s/c ≈ 15) were required.

A similar procedure was claimed in GB 788,301. MHA was ethynylated in ether in presence of ammonia using sodium and ethyne followed by Lindlar hydrogenation in ligroin as solvent. The ethynylation was described to proceed in 82.2 % yield.

I. N. Nazarov et al. described in Seriya Khimicheskaya 1957, 1267-1270 and in Zhurnal Obshchei Khimii 1958, 28, 1444-1448 the semi hydrogenation of alkynols in presence of Pd on CaCO₃. MHA was ethynylated in 87% yield in presence of solid KOH at 0-20°C at 5-8 atmospheres, followed by hydrogenation (90%), no conditions were given.

A. Ofner et al. described in Helv. Chim. Acta 1959,42, 2577-2584 the Pd/CaCO₃ (5% Pd loading) catalyzed hydrogenation of MH to MHA at 34 atm pressure, followed by sodium acetylide treatment to give the corresponding alkynol and Lindlar hydrogenation to DMOE (named as dihydro-linalool).

F. J. Bröcker et al. claimed the preparation and application of hydrogenation catalysts. In EP-A 412 415 the preparation of a Pd catalyst on a support by metal vapour deposition was claimed (300-800°C), and especially the hydrogenation of 3,7-dimethyl-oct-1-in-3-ol (named as hydro-dehydrolinalool (HDHL)) to DMOE (named as hydro-linalool (HLIN)) was demonstrated (examples 2 and 3: 100% conversion, 99.3% - 99.5% selectivity).

EP-A 754 664 describes the partial hydrogenation of alkynes using a solid-bed catalyst as described in EP-A 412 415, whereby the Lindlar hydrogenation was carried out in presence of CO (10-180 ppm).

In EP-A 1 110 932 the above applications are combined and more examples are presented. A similar procedure of alkyne hydrogenation in presence of CO was carried out.

EP-A 816 321 claimed the synthesis of MHA by aldol reaction of acetone and isovaleraldehyde in presence of hydrogen and a conventional hydrogenation catalyst at 15-150°C and 1-100 bar, (example 3: Pd/C). For the ethynylation ofMHA standard protocols were used (example 3, step c, 2 hours, 4-6°C, KOH in water, ammonia). Solvents like NMP, DMSO, and DMF had to be used. These solvents are difficult to separate from the product, (and) especially they could have a negative impact on flavour and fragrance applications. The Lindlar hydrogenation was carried out in a solvent, e.g. hexane, heptane, at 1-130°C and at 1 -50 bar. The preferred catalyst was Pd on CaCO₃ (selectivity 95-96 %, conversion 97.3-99.7%). In the examples (step 3c, page 20) the Lindlar hydrogenation was carried out in hexane.
The object of the present invention was to provide a process which may be used for the industrial production of DMOE, i.e. a process which is economic. Furthermore, the process according to the present invention should not have the disadvantages of the processes of the prior art.
A preferred object of the present invention was also to achieve a selectivity > 90% at a conversion of > 95%.
The present invention is directed to a process for the manufacture of 3,7-dimethyl-1-octen-3-ol comprising the following steps:
a) hydrogenation of 6-methyl-5-hepten-2-on to 6-methyl-2-heptanon in the presence of hydrogen and a palladium containing catalyst on a carrier selected from the group consisting of carbon, calcium carbonate and aluminum oxide.
b) reaction of 6-methyl-2-heptanon with acetylene to 3,7-dimethyl-1-octin-3-ol in the presence of ammonia and potassium hydroxide and in the absence of any additional organic solvent;
c) hydrogenation of 3,7-dimethyl-1-octin-3-ol to 3,7-dimethyl-1-octen-3-ol in the presence of hydrogen and a palladium containing catalyst on a carrier selected from the group consisting of calcium carbonate, aluminum oxide, silica, porous glass, carbon or graphite, and barium sulphate, with the proviso that the catalyst additionally contains lead when the carrier is calcium carbonate;
wherein step a) is carried out at a pressure in the range of 1.1 bar to 15 bar, and wherein step a) and step c) are performed in the absence of any organic solvent.

Big advantages of the process of the present invention are that the same catalyst as used in step a) may be used in step c), and that steps a) and c) are performed without the use of an organic solvent such as petroleum ether, ligroin, hexane and heptane.

The single steps are described in more detail in the following.

### Step a)

Step a) is performed in the absence of any organic solvent, i.e. that no organic solvent is added to MH or the reaction mixture used in step a). This encompasses also the case that minor amounts of solvent may be present as "impurity" of the MH used.

Preferably the catalyst used in step a) is the same catalyst as used in step c). More preferably this catalyst has a Pd content, based on the total weight of the catalyst, of 1-10, preferred 2.5-8 more preferred, especially preferred 3-7 weight-% Pd. This catalyst is even more preferably Pd on aluminum oxide.

If the catalyst is Pd on aluminum oxide, it preferably has a BET surface area in the range of 50 to 500 m²/g, more preferably it has a BET surface area in the range of 80 to 300 m²/g, most preferably it is an egg-shell catalyst.

An "egg-shell" catalyst in the context of the present invention is a catalyst where the catalytically active metal (Pd) has a non-uniform distribution on the support and is located mainly on the shell of such catalyst.

If the catalyst is Pd on carbon, it preferably has a BET surface area in the range of 800 to 1500 m²/g, more preferably it has a BET surface area in the range of 900-1200 m²/g. Even more preferably 50% of the catalyst also have a size ≤ 20-50 µm (i.e. the so-called particle size D50 ≤ 20-50 µm). Most preferably the bulk density is in the range of 100 to 500 g/L, more preferably in the range of 200 to 300 g/L.

If the catalyst is Pd on CaCO₃, it preferably has a BET surface area in the range of 5 to 15 m²/g, more preferably it has a BET surface area in the range of 7 to 10 m²/g. Most preferably 50% of the particles of this catalyst also have a size ≤ 3-30 µm (i.e. the so-called particle size D50 ≤ 3-30 µm).

Even more preferably pulverous catalysts including egg-shell catalysts are used. These can be separated easily after the reaction from the reaction mixture by filtration or centrifugation. Furthermore, these catalysts can be recycled and used several times.

Preferably step a) is carried out at a temperature in the range of 20 to 100 °C, more preferably at a temperature in the range of 25 to 80 °C.

Step a) is carried out at a pressure in the range of 1.1 bar to 15 bar, preferably at a pressure in the range of 1.5 to 6 bar, more preferably at a pressure in the range of 1.8 to 4 bar.

Preferably the amount in weight-% of catalyst used in step a) is in the range of 1 : 50 to 1 : 5000, more preferably in the range of 1 : 100 to 1 : 2500, even more preferably in the range of 1 : 250 to 1 : 1000, based on the amount of 6-methyl-5-hepten-2-on in weight-%.

Preferred embodiments of the present invention are also embodiments where several preferred embodiments of step a) as listed above are combined.

### Step b)

Preferably the molar ratio of 6-methyl-2-heptanon to acetylene in step b) is in the range of 1 : 1 to 1 : 2, more preferably in the range of 1 : 1.01 to 1 : 1.5.

Preferably the molar ratio of 6-methyl-2-heptanon to potassium hydroxide in step b) is in the range of 30 : 1 to 250 : 1, more preferably in the range of 40 : 1 to 100 : 1.

Preferably the molar ratio of 6-methyl-2-heptanon to ammonia in step b) is in the range of 1 : 15 to 1 : 50, more preferably in the range of 1 : 20 to 1 : 30.

Preferably step b) is carried out at a temperature in the range of-10 °C to 25 °C, more preferably at a temperature in the range of 0 to 20 °C. The pressure used is preferably in the range of 5 to 25 bar, more preferably in the range of 10 to 20 bar.

Preferred embodiments of the present invention are also embodiments where several preferred embodiments of step b) as listed above are combined.

### Step c)

Step c) is performed in the absence of any organic solvent, i.e. that no organic solvent is added to DMOI or the reaction mixture used in step c). This encompasses also the case that minor amounts of solvent may be present as "impurity" of the DMOI used.

Preferably the catalyst used in step c) is a palladium containing catalyst on a carrier selected from the group consisting of calcium carbonate, aluminum oxide, silica, porous glass (especially TRISOPERL®), with the proviso that the catalyst additionally contains lead when the carrier is calcium carbonate.

Preferably the catalyst used in step c) has an amount of palladium in the range of 1 to 10 weight-%, more preferably in the range of 1 to 5 weight-%, based on the total weight of the catalyst, if the carrier is aluminum oxide, silica, porous glass, carbon or graphite and barium sulphate.

A preferred catalyst used in step c) is palladium on calcium carbonate, wherein lead is present. Preferably this catalyst has an amount of lead in the range of 0 to 9 weight-%, more preferably in the range of 1 to 5 weight-%, based on the total weight of the catalyst. The amount of palladium of this catalyst is preferably in the range of 1 to 10 weight-%, more preferably in the range of 3 to 8 weight-%, even more preferably in the range of 5 to 7 weight-%, based on the total weight of the catalyst,

If the catalyst is Pd + Pb on CaCO₃, it preferably has a BET surface area in the range of 2 to 15 m²/g, more preferably it has a BET surface area in the range of 5 to 15 m²/g, even more preferably it has a BET surface area in the range of 7 to 10 m²/g. Most preferably 50% of the particles of this catalyst also have a size ≤ 2 to 50 µm (i.e. the so-called particle size D50 ≤ 3-30 µm), preferably ≤ 2 to 30 µm, more preferably ≤ 3 to 10 µm.

Most preferably, however, the catalyst used in step c) is the same catalyst as used in step a). This catalyst is more preferably Pd on aluminum oxide.

If the catalyst is Pd on aluminum oxide or silica or any mixture thereof it preferably has a BET surface area in the range of 50 to 500 m²/g, more preferably it has a BET surface area in the range of 80 to 300 m²/g, most preferably it is an egg-shell catalyst.

Preferably step c) is carried out at a temperature in the range of 10 °C to 100 °C, more preferably at a temperature in the range of 15 to 60 °C.

Preferably step c) is carried out at a pressure in the range of 1.1 bar to 15 bar, more preferably at a pressure in the range of 1.5 to 6 bar, even more preferably at a pressure in the range of 1.8 to 4 bar.

Preferably the amount in weight-% of the catalyst used in step c) is in the range of 1 : 50 to 1 : 5000, more preferably in the range of 1 : 100 to 1 : 2500, even more preferably in the range of 1 : 250 to 1 : 1000, based on the amount of 3,7-dimethyl-1-octin-3-ol in weight-%.

Preferred embodiments of the present invention are also embodiments where several preferred embodiments of step c) as listed above are combined.

Further preferred embodiments of the present invention are embodiments where one or more of the preferred embodiments of step a) and/or step b) and/or step c) as listed above are combined.

The present invention is not only directed to the synthesis of DMOE, but also to the synthesis of (iso)phytol (derivatives) and vitamin E (acetate), especially to
◊ a process for the manufacture of isophytol comprising the following steps
   - preparing DMOE according to the process of the present invention;
   - preparing 6,10-dimethyl-5-undecen-2-one starting from a thus prepared DMOE;
   - hydrogenating a thus prepared 6,10-dimethyl-5-undecen-2-one to obtain hexahydropseudoionone;
   - preparing (iso)phytol or derivatives thereof from a thus prepared hexahydropseudoionone.
   as well as to
◊ a process for the manufacture of vitamin E (acetate) comprising the following steps
   - preparing DMOE according to the process of the present invention;
   - preparing 6,10-dimethyl-5-undecen-2-one starting from a thus prepared DMOE;
   - hydrogenating a thus prepared 6,10-dimethyl-5-undecen-2-one to obtain hexahydropseudo ionone;
   - preparing (iso)phytol or derivatives thereof from a thus prepared hexahydropseudoionone;
   - reacting 2,3,6-trimethylhydroquinone (TMHQ) or 2,3,6-trimethylhydroquinone-l-acetate (TMHQA) with (iso)phytol or derivatives thereof or any mixture thereof to obtain vitamin E or its acetate.

Derivatives of phytol and isophytol are especially those as disclosed in EP-A 694-541: page 4, line 39 to page 5, line 14 and in WO 2005/121115: page 4, line 11 to 19 with n = 3.

The invention is now further illustrated in the following non-limiting examples.

### Examples

In the examples the following catalysts were used:
- a 5 % Pd/C catalyst which was oxidized and dried and is e.g. commercially available from Evonik under the tradename "5 % Pd/C E 101 O/D" ("**catalyst A**");
- a 5 % Pd/C catalyst with a BET surface area of 1000 m²/g, a bulk density in the range of 200 and 300 g/L and the following particle size distribution: 10 % of the particles ≤ 6 µm, 50 % of the particles ≤ 28 µm, and 90 % of the particles ≤ 79 µm, which is e.g. commercially available from Evonik under the tradename "5 % Pd/C E 101 N/D" ("**catalyst B**");
- a 5 % Pd/C catalyst with a specific surface area of 1000 m²/g, a volume of the micropores of 0.30 ml/g, a volume of the mesopores of 0.40 ml/g, a volume of the macropores of 0.40 ml/g, a bulk density in the range of 200 and 300 g/L; a volume of the pores of 1.10 ml/g and a mixed metal localisation, which is e.g. commercially available from Evonik under the tradename "10 % Pd/C Evonik E 101 N/D" ("**catalyst C**");
- a 5 % Pd/CaCO₃ egg-shell catalyst with a BET surface area of 8 m²/g, a bulk density of 0.37 kg/l, and whereby 50 % of the particles have a size ≤ 5 µm which is e.g. commercially available from Evonik under the tradename "5 % Pd/CaCO₃ Evonik E 407 R/D" ("**catalyst D**");
- a 5 % Pd/Al₂O₃ egg-shell catalyst with a surface area in the range of 50 - 300 m²/g, a bulk density in the range of 0.5 - 1 kg/l and a pore volume in the range of 0.1 to 0.5 ml/g. Such a catalyst with a surface area of 93 m²/g, a bulk density of 0.8 kg/l and a pore volume of 0.3 ml/g is e.g. commercially available from Evonik under the tradename "5 % Pd/Al₂O₃ Evonik E 213 XR/D" ("**catalyst E**");
- a 5 % Pd/Al₂O₃ catalyst as e.g. commercially available from Engelhard under the tradename "5 % Pd/Al₂O₃ Engelhard Lot. 09784" ("**catalyst F**");
- a 5 % Pt/C catalyst with a BET area in the range of 500 to 1000 m²/g, whereby 50 % of the particles have a size ≤ 21 µm. Such a catalyst with a BET area of 800 m²/g, whereby 50 % of the particles have a size ≤ 21 µm, is e.g. commercially available from Engelhard under the tradename "5 % Pt/C Engelhard Lot. 07608" ("**catalyst G**");
- a 5 % Rh/C catalyst which is e.g. commercially available from Evonik under the tradename "5 % Rh/C, Evonik G 101 XB/D" ("**catalyst H**");
- a catalyst with 5 % Pd and 3.5 % Pb supported on CaCO₃ with a BET area in the range of 5 to 15 m²/g and a bulk density in the range of 0.2 to 1.0 kg/l, whereby 50 % of the particles have a size ≤ 2 to 10 µm. Such a catalyst with a BET area of 8 m²/g and a bulk density of 0.37 kg/l, whereby 50 % of the particles have a size ≤ 5 µm is e.g. commercially available from Evonik under the tradename "5 % Pd 3.5% Pb CaCO₃ Evonik CE 407 R/D" ("**catalyst I**");
- a catalyst with 5 % Pd and 1 % Pb supported on CaCO₃ as e.g. commercially available from J & M under the tradename "5 % Pd 1% Pb CaCO₃ J & M A-304050-5" ("**catalyst J**");
- a catalyst with 7 % Pd and 9 % Pb supported on CaCO₃ as e.g. commercially available from Evonik under the tradename "7 % Pd 9 % Pb CaCO₃ Evonik CE 407 R/D" ("**catalyst K**");
- an egg-shell catalyst with 5 % Pd and 2.5 % Pb supported on CaCO₃ with a BET area in the range of 5 to 15 m²/g, whereby 50 % of the particles have a size ≤ 20 to 50 µm which may be manufactured according to processes known to the person skilled in the art ("**catalyst L**");
- a catalyst with 5 % Pd and 5 % Pb supported on CaCO₃ with a BET area in the range of 2 to 15 m²/g, a bulk density in the range of 0.2 to 1.0 kg/l and a pore volume in the range of 1.0 to 2.0 ml/g, whereby the particles have a size ≤ 100 µm. Such a catalyst with a BET area of 5 m²/g, a bulk density of 0.45 kg/L and a pore volume of 1.6 ml/g, whereby the particles have a size ≤ 100 µm is e.g. commercially available from Heraeus under the tradename "5 % Pd 5 % Pb CaCO₃ Heraeus"("**catalyst M**");
- a 5 % Pd/SiO₂ catalyst as e.g. commercially available from Engelhard under the tradename "5 % Pd/SiO₂ Engelhard A 596033" ("**catalyst N**");
- a 1 % Pd/C catalyst as e.g. commercially available from Evonik under the tradename "1 % Pd/charcoal Degussa E 101 N/D" ("**catalyst O**");
- a 5 % Pd/graphite reduced catalyst as e.g. commercially available from Engelhard under the tradename "5 % Pd/graphite Engelhard" ("**catalyst P**");
- a 5 % Pd/BaSO₄ catalyst as e.g. commercially available from Evonik under the tradename "5 % Pd/BaSO₄ Evonik E 50 N/D" ("**catalyst Q**");
- a 3.7 weight-% Pd/Al₂O₃ catalyst, whereby the Pd is in the form ofnano-particles as e.g. commercially available from SDC under the tradename "3.7 % Pd/Al₂O₃ SDC Nanokat" ("**catalyst R**");
- a 1 % Pd/TP catalyst ("**catalyst S**") whose manufacture is described below.

### 1 % Pd/TP was manufactured as follows:

21 mg Pd(OAc)₂ (0,09 mmol) were suspended in 50 mL of dichloromethane. 1 g of TRISOPERL® were added and the solvent was removed (bath temperature: 40 °C / pressure: 950 mbara). The carrier doped with Pd(OAc)₂ was calcinated for 2 hours at 300 °C in an oven (pre-heating of the oven for 20 minutes for 1000 W to 300 °C). The loading of the catalyst on the carrier was then ca. 1 weight-% Pd, i.e. 10 mg of Pd onl g of carrier. TRISOPERL® by the Schuller GmbH, Wertheim/Germany, is a porous Silica glass with an average particle size in the range of 100 to 200 µm, an average pore size of 54.47 nm, a specific surface of 93.72 m²/g and an average pore volume of 1255.5 mm³/g.

### Step a: Examples 1 to 7

A 125 mL-autoclave (Hastelloy) was charged with 30 g of MH with a purity of 96.7 % and the catalyst as given in table 1. The amount of catalyst was 33.3 mg in all examples. The mixture was heated to 60 °C and hydrogen was added at 2 bar. After reaction, when no up-take of hydrogen was observed any more, the mixture was cooled to 20 °C, the catalyst separated by filtration and the mixture analyzed by gas chromatography.

**Table 1: Synthesis of MHA by use of various catalysts**

| **Example** | **Catalyst** | **Reaction time [h** : **min]** | **Conversion [mol%]** | **Selectivity [mol%]** | **Yield [mol%]** |
|---|---|---|---|---|---|
| 1 | **A**: 5% Pd/C | 11 : 40 | 99.2 | 97.9 | 97.1 |
| 2 | **B:** 5 % Pd/C | 21 : 00 | 98.6 | 87.7 | 86.5 |
| 3 | **D:** 5 % Pd/CaCO₃ | 24 : 00 | 95.4 | 97.6 | 93.2 |
| 4 | **C:** 10 % Pd/C | 43 : 34 | 94.3 | 97.8 | 92.3 |
| 5 | **E:** 5 % Pd/Al₂O₃ | 66 : 00 | 100.0 | 97.3 | 97.3 |
| 6 | **F**: 5 % Pd/Al₂O₃ | 24 : 00 | 80.5 | 89.6 | 72.1 |
| 7 | **R:** 3.7 % Pd/Al₂O₃ | 39 : 46 | 63.4 | 88.6 | 56.2 |

The catalysts used in examples 5 and 6 are pulverous catalysts.

### Step a: Examples 8 to 11

Example 2 was repeated at various temperatures and/or pressures. The results are shown in table 2. The catalyst used was catalyst **B** (5 % Pd/C).

**Table 2: Synthesis of MHA at various temperatures and/or pressures**

| **Example** | **Amount of catalys t [mg]** | **Temperature [°C]** | **Pressure [bar]** | **Reaction time [h : min]** | **Conversion [mol%]** | **Selectivity [mol%]** | **Yield [mol% ]** |
|---|---|---|---|---|---|---|---|
| 2 | 33.3 | 60 | 2 | 21 : 00 | 98.6 | 87.7 | 86.5 |
| 8 | 33.3 | 30 | 2 | 24 : 00 | 99.8 | 93.5 | 93.4 |
| 9 | 33.3 | 80 | 2 | 24 : 00 | 62.1 | 80.0 | 49.7 |
| 10 | 33.3 | 60 | 10 | 24 : 00 | 99.8 | 94.2 | 94.0 |
| 11 | 33.3 | 30 | 10 | 20 : 00 | 99.9 | 91.9 | 91.8 |

### Step a: Comparison examples 1 and 2

Example 1 was repeated but different metals used as catalyst. The results are summarized in table 3. As the results show platinum on carbon as well as rhodium on carbon give less conversion and less selectivity, thus also less yield on the desired product, than palladium on carbon.

**Table 3: Synthesis of MHA by use of different metals on carbon as catalyst**

| **Example** | **Catalyst** | **Amount of catalyst [mg]** | **Reaction time [h : min]** | **Conversion [mol%]** | **Selectivity [mol%]** | **Yield [mol%]** |
|---|---|---|---|---|---|---|
| 1 | **A**: 5 % Pd/C | 33.3 | 11 : 40 | 99.2 | 97.9 | 97.1 |
| Comparison example 1 | **G:** 5 % Pt/C | 33.3 | 24 : 00 | 39.4 | 74.2 | 29.2 |
| Comparison example 2 | **H**: 5 % Rh/C | 33.3 | 24 : 00 | 54.0 | 85.8 | 46.3 |

### Step b: Example 12

In a 2 liter autoclave under nitrogen, 860 g of liquid ammonia were added to 319.4 g of methylheptanone. The mixture was cooled down to 15 °C (pressure: 9.8 bar). Then acetylene was added until a pressure of 12 bar was achieved. Then 6.4 g of an aqueous 45 weight-% KOH solution was added. The reactor pressure of 12 bar was maintained by continued addition of acetylene. In total 66.7 g of acetylene were used. At the end of the reaction (after ca. 3.3 hours) 10 g of an aqueous 90 weight-% acetic acid solution were added, the pressure was relieved whereby the main part of ammonia was evaporated. By heating to 40 °C the remaining ammonia was removed. Then the reaction mixture was washed twice with 100 ml of de-ionized water, twice with 100 ml of an aqueous 8 weight-% sulphuric acid solution and again twice with 100 ml of de-ionized water.

The experiment was repeated three times. The conversion was 97.3 % in average, the yield was 96.0 % in average before washing and 95.3 % afterwards, and the selectivity was 98.7 %.

### Step c: Examples 13 to 30

A 150 mL-autoclave (Hastelloy) was charged with 40 g of DMOI with a purity of 99.8 % and the amount and catalyst as given in table 4. 4.7 mg of the modifier 2,2'ethylene-dithiodiethanol were added. The mixture was heated to 30 °C and hydrogen was added at 2 bar. After reaction, when no up-take of hydrogen was observed any more, the mixture was cooled to 20 °C, the catalyst separated by filtration and the mixture analyzed by gas chromatography.

**Table 4: Synthesis of DMOE by use of various catalysts**

| **Example** | **Catalyst** | **Amount of catalyst [mg]** | **Reaction time [h : min]** | **Conversion [mol%]** | **Selectivity [mol%]** | **Yield [mol%]** |
|---|---|---|---|---|---|---|
| 13 | **I**: 5% Pd 3.5% Pb CaCO₃ | 34.8 | 16 : 00 | 99.5 | 92.3 | 91.9 |
| 14 | **I**: 5% Pd 3.5% Pb CaCO₃ | 34.8 | 05 : 49 | 100.0 | 94.2 | 94.2 |
| 15 | **I**: 5% Pd 3.5% Pb | 34.8 | 04 : 44 | 99.5 | 95.4 | 94.1 |
| | CaCO₃ | | | | | |
| 16 | **I**: 5% Pd 3.5% Pb CaCO₃ | 34.8 | 04 : 42 | 100.0 | 95.2 | 95.2 |
| 17 | **J:** 5% Pd 1% Pb CaCO₃ | 34.8 | 03 : 49 | 98.5 | 92.7 | 93.9 |
| 18 | **J:** 5% Pd 1% Pb CaCO₃ | 24.8 | 03 : 51 | 100.0 | 92.8 | 92.8 |
| 19 | **K:** 7% Pd 9% Pb CaCO₃ | 34.8 | 04 : 20 | 99.4 | 94.1 | 95.4 |
| 20 | **L**: 5% Pd 2.5% Pb CaCO₃ | 34.8 | 06 : 25 | 99.5 | 95.3 | 96.5 |
| 21 | **M:** 5% Pd 5% Pb CaCO₃ | 34.8 | 08 : 00 | 99.4 | 95.5 | 96.8 |
| 22 | **S:** 1% Pd/Trisoperl | 180.0 | 04 : 06 | 98.9 | 91.0 | 88.5 |
| 23 | **S**: 1% Pd/Trisoperl | 120.0 | 04 : 37 | 98.3 | 91.7 | 87.7 |
| 24 | **E**: 5% Pd/Al₂O₃ | 34.8 | 04 : 01 | 98.6 | 90.9 | 92.1 |
| 25 | **E**: 5% Pd/Al₂O₃ | 20.0 | 04 : 31 | 98.6 | 90.6 | 91.8 |
| 26 | **N**: 5% | 34.8 | 23 : 03 | 100.0 | 81.9 | 83.0 |
| | Pd/SiO₂ | | | | | |
| 27 | **O**: 1% Pd/charcoal | 34.8 | 16 : 52 | 98.4 | 88.4 | 84.7 |
| 28 | **P**: 5% Pd/graphite | 34.8 | 21 : 00 | 97.6 | 88.4 | 82.9 |
| 29 | **F**: 5% Pd/Al₂O₃ | 34.8 | 20 : 30 | 98.8 | 89.4 | 86.6 |
| 30 | **Q**: 5% Pd/BaSO₄ | 69.6 | 5 : 00 | 98.5 | 90.4 | 86.8 |

### Step c: Examples 31 to 36

Example 13 was repeated at different temperature and/or pressure. The results are shown in table 5. The catalyst used was catalyst **I** (5 % Pd 3.5% Pb on CaCO₃).

**Table 5: Synthesis of DMOE at different temperature and/or pressure**

| **Exampl e** | **Amou nt of catalys t [mg]** | **Temperatu re [°C]** | **Pressur e [bar]** | **Reactio n time [h : min]** | **Conversio n [mol%]** | **Selectivit y [mol%]** | **Yield [mol% ]** |
|---|---|---|---|---|---|---|---|
| 13 | 34.8 | 30 | 2 | 16 : 00 | 99.5 | 92.3 | 91.9 |
| 31 | 34.8 | 30 | 2 | 25 : 00 | 100.0 | 93.1 | 93.1 |
| 32 | 34.8 | 30 | 2 | 6 : 30 | 99.9 | 94.6 | 94.2 |
| 33 | 34.8 | 40 | 2 | 21 : 30 | 100.0 | 91.4 | 91.4 |
| 34 | 34.8 | 40 | 2 | 10 : 45 | 100.0 | 91.7 | 91.7 |
| 35 | 34.8 | 20 | 2 | 22 : 00 | 100.0 | 93.8 | 93.8 |
| 36 | 34.8 | 30 | 10 | 22 : 00 | 100.0 | 90.0 | 90.0 |

### Step c: Examples 37 and 38

Example 13 was repeated with different modifiers. The reactions were all performed at 2 bar and 30 °C. The results are shown in table 6.

**Table 6: Synthesis of DMOE, whereby the catalysts were modified with different modifiers**

| **Example** | **Modifier** | **Amount of modifier [mg]** | **Reaction time [h : min]** | **Conversion [mol%]** | **Selectivity [mol%]** | **Yield [mol%]** |
|---|---|---|---|---|---|---|
| 13 | 2,2'ethylene-dithiodiethanol | 4.7 | 16 : 00 | 99.5 | 92.3 | 91.9 |
| 37 | Thiophene | 4.7 | 8 : 10 | 100.0 | 89.7 | 89.7 |
| 38 | Pyridine | 4.7 | 5 : 30 | 100.0 | 89.2 | 89.2 |

### Step c: Example 39 and comparison examples 40 to 41

Example 13 was repeated with different metal catalysts, but no modifier was used. The reactions were all performed at 2 bar and 30 °C. The results are shown in table 7.

**Table 7: Synthesis of DMOE with different catalysts, whereby no modifier was used**

| **Example** | **Catalyst** | **Amount of catalyst [mg]** | **Reaction time [h : min]** | **Conversion [mot%]** | **Selectivity [mol%]** | **Yield [mol%]** |
|---|---|---|---|---|---|---|
| 39 | **I**: 5% Pd 3.5% Pb CaCO₃ | 34.8 | 5 : 38 | 100.0 | 91.2 | 91.2 |
| Comparison example 40 | **G**: 5% Pt/C | 34.8 | 19 : 00 | 89.4 | 51.4 | 37.3 |
| Comparison example 41 | **H**: 5% Rh/C | 34.8 | 24 : 00 | 71.8 | 60.8 | 16.4 |

## Claims

1. A process for the manufacture of 3,7-dimethyl-1-octen-3-ol comprising the following steps:
a) hydrogenation of 6-methyl-5-hepten-2-on to 6-methyl-2-heptanon in the presence of hydrogen and a palladium containing catalyst on a carrier selected from the group consisting of carbon, calcium carbonate and aluminum oxide;
b) reaction of 6-methyl-2-heptanon with acetylene to 3,7-dimethyl-1-octin-3-ol in the presence of ammonia and potassium hydroxide and in the absence of any additional organic solvent;
c) hydrogenation of 3,7-dimethyl-1-octin-3-ol to 3,7-dimethyl-1-octen-3-ol in the presence of hydrogen and a palladium containing catalyst on a carrier selected from the group consisting of calcium carbonate, aluminum oxide, silica, porous glass, carbon or graphite, and barium sulphate, with the proviso that the catalyst additionally contains lead when the carrier is calcium carbonate;
wherein step a) is carried out at a pressure in the range of 1.1 bar to 15 bar, and wherein step a) and step c) are performed in the absence of any organic solvent.

2. The process according to claim 1, wherein the catalyst used in step a) is the same catalyst as used in step c).

3. The process according to claim 2, wherein the catalyst is Pd on aluminum oxide.

4. The process according to claim 1, wherein the catalyst used in step c) is palladium on calcium carbonate, wherein lead is present.

5. The process according to any of the preceding claims, wherein step a) is carried out at a temperature in the range of 20 to 100°C, and/or at a pressure in the range of 1.5 to 6 bar.

6. The process according to any of the preceding claims, wherein the amount in weight-% of catalyst used in step a) is in the range of 1 : 50 to 1 : 5000, based on the amount of 6-methyl-5-hepten-2-on in weight-%.

7. The process according to any of the preceding claims, wherein the molar ratio of 6-methyl-2-heptanon to acetylene in step b) is in the range of 1 : 1 to 1 : 2, and/or wherein the molar ratio of 6-methyl-2-heptanon to potassium hydroxide in step b) is in the range of 30 : 1 to 250 : 1, and/or wherein the molar ratio of 6-methyl-2-heptanon to ammonia in step b) is in the range of 1 : 15 to 1 : 50.

8. The process according to any of the preceding claims, wherein step b) is carried out at a temperature in the range of-10°C to 25 °C, and at a pressure in the range of 5 to 25 bar.

9. The process according to any of the preceding claims, wherein step c) is carried out at a temperature in the range of 20°C to 100°C, and/or at a pressure in the range of 1.1 bar to 10 bar.

10. The process according to any of the preceding claims, wherein the amount in weight-% of the catalyst used in step c) is in the range of 1 : 50 to **1** : 5000, based on the amount of 3,7-dimethyl-1-octin-3-ol in weight-%.

11. The process according to any of the preceding claims, wherein the catalyst used in step c) has an amount of palladium in the range of 1 to 10 weight-%, based on the total weight of the catalyst.

12. The process according to claim 4, wherein the catalyst used in step c) has an amount of lead in the range of 0 to 9 weight-%, based on the total weight of the catalyst.

13. A process for the manufacture of phytol or isophytol comprising the following steps
- preparing 3,7-dimethyl-1-octen-3-ol according to any of the preceding claims;
- preparing 6,10-dimethyl-5-undecen-2-one starting from a thus prepared 3,7-dimethyl-1-octen-3-ol;
- hydrogenating a thus prepared 6,10-dimethyl-5-undecen-2-one to obtain hexahydropseudoionone;
- preparing phytol or isophytol from a thus prepared hexahydropseudoionone.

14. A process for the manufacture of vitamin E or its acetate comprising the following steps:
- preparing 3,7-dimethyl-1-octen-3-ol according to any of claims 1 to 12;
- preparing 6,10-dimethyl-5-undecen-2-one starting from a thus prepared 3,7-dimethyl-1 -octen-3 -ol;
- hydrogenating a thus prepared 6,10-dimethyl-5-undecen-2-one to obtain hexahydropseudoionone;
- preparing phytol or isophytol from a thus prepared hexahydropseudoionone;
- reacting 2,3,6-trimethylhydroquinone or 2,3,6-trimethylhydroquinone-1-acetate with phytol or isophytol or any mixture thereof to obtain vitamin E or its acetate.

## Patentansprüche

1. Verfahren zur Herstellung von 3,7-Dimethyl-1-octen-3-ol, umfassend folgende Schritte:
a) Hydrieren von 6-Methyl-5-hepten-2-on zu 6-Methyl-2-heptanon in Gegenwart von Wasserstoff und eines palladiumhaltigen Katalysators auf einem Träger ausgewählt aus der Gruppe bestehend aus Kohle, Calciumcarbonat und Aluminiumoxid;
b) Umsetzen von 6-Methyl-2-heptanon mit Acetylen zu 3,7-Diméthyl-1-octin-3-ol in Gegenwart von Ammoniak und Kaliumhydroxid und ohne Vorhandensein eines zusätzlichen organischen Lösungsmittels;
c) Hydrieren von 3,7-Diméthyl-1-octin-3-ol zu 3,7-Dimethyl-1-octen-3-ol in Gegenwart von Wasserstoff und eines palladiumhaltigen Katalysators auf einem Träger ausgewählt aus der Gruppe bestehend aus Calciumcarbonat, Aluminiumoxid, Silica, porösem Glas, Kohle oder Graphit und Bariumsulfat, mit der Maßgabe, dass der Katalysator zusätzlich Blei enthält, wenn der Träger Calciumcarbonat ist;
wobei Schritt a) bei einem Druck in dem Bereich von 1,1 bar bis 15 bar durchgeführt wird und wobei Schritt a) und Schritt c) ohne Vorhandensein eines organischen Lösungsmittels durchgeführt werden.

2. Verfahren gemäß Anspruch 1, wobei der bei Schritt a) verwendete Katalysator der gleiche Katalysator wie bei Schritt c) verwendet ist.

3. Verfahren gemäß Anspruch 2, wobei der Katalysator Pd auf Aluminiumoxid ist.

4. Verfahren gemäß Anspruch 1, wobei der bei Schritt c) verwendete Katalysator Palladium auf Calciumcarbonat ist, wobei Blei vorhanden ist.

5. Verfahren gemäß einem der vorstehenden Ansprüche, wobei Schritt a) bei einer Temperatur in dem Bereich von 20 bis 100 °C und/oder bei einem Druck in dem Bereich von 1,5 bis 6 bar durchgeführt wird.

6. Verfahren gemäß einem der vorstehenden Ansprüche, wobei die bei Schritt a) verwendete Menge an Katalysator in Gew.-% in dem Bereich von 1:50 bis 1:5000 bezogen auf die Menge an 6-Methyl-5-hepten-2-on in Gew.-% beträgt.

7. Verfahren gemäß einem der vorstehenden Ansprüche, wobei das Molverhältnis von 6-Methyl-2-heptanon zu Acetylen bei Schritt b) in dem Bereich von 1:1 bis 1:2 liegt und/oder wobei das Molverhältnis von 6-Methyl-2-heptanon zu Kaliumhydroxid bei Schritt b) in dem Bereich von 30:1 bis 250:1 liegt und/oder wobei das Molverhältnis von 6-Methyl-2-heptanon zu Ammoniak bei Schritt b) in dem Bereich von 1:15 bis 1:50 liegt.

8. Verfahren gemäß einem der vorstehenden Ansprüche, wobei Schritt b) bei einer Temperatur in dem Bereich von -10 °C bis 25 °C und bei einem Druck in dem Bereich von 5 bis 25 bar durchgeführt wird.

9. Verfahren gemäß einem der vorstehenden Ansprüche, wobei Schritt c) bei einer Temperatur in dem Bereich von 20 °C bis 100 °C und/oder bei einem Druck in dem Bereich von 1,1 bar bis 10 bar durchgeführt wird.

10. Verfahren gemäß einem der vorstehenden Ansprüche, wobei die bei Schritt c) verwendete Menge an Katalysator in Gew.-% in dem Bereich von 1:50 bis 1:5000 bezogen auf die Menge an 3,7-Dimethyl-1-octin-3-ol in Gew.-% beträgt.

11. Verfahren gemäß einem der vorstehenden Ansprüche, wobei der bei Schritt c) verwendete Katalysator eine Menge an Palladium in dem Bereich von 1 bis 10 Gew.-% bezogen auf das Gesamtgewicht des Katalysators aufweist.

12. Verfahren gemäß Anspruch 4, wobei der bei Schritt c) verwendete Katalysator eine Menge an Blei in dem Bereich von 0 bis 9 Gew.-% bezogen auf das Gesamtgewicht des Katalysators aufweist.

13. Verfahren zur Herstellung von Phytol oder Isophytol, umfassend folgende Schritte
- Herstellen von 3,7-Dimethyl-1-octen-3-ol gemäß einem der vorstehenden Ansprüche;
- Herstellen von 6,10-Dimethyl-5-undecen-2-on ausgehend von einem so hergestellten 3,7-Dimethyl-1-octen-3-ol;
- Hydrieren eines so hergestellten 6,10-Dimethyl-5-undecen-2-ons, um Hexahydropseudoionon zu erhalten;
- Herstellen von Phytol oder Isophytol aus einem so hergestellten Hexahydropseudoionon.

14. Verfahren zur Herstellung von Vitamin E oder seinem Acetat, umfassend folgende Schritte
- Herstellen von 3,7-Dimethyl-1-octen-3-ol gemäß einem der Ansprüche 1 bis 12;
- Herstellen von 6,10-Dimethyl-5-undecen-2-on ausgehend von einem so hergestellten 3,7-Dimethyl-1-octen-3-ol;
- Hydrieren eines so hergestellten 6,10-Dimethyl-5-undecen-2-ons, um Hexahydropseudoionon zu erhalten;
- Herstellen von Phytol oder Isophytol aus einem so hergestellten Hexahydropseudoionon;
- Umsetzen von 2,3,6-Trimethylhydrochinon oder 2,3,6-Trimethylhydrochinon-1-acetat mit Phytol oder Isophytol oder einem Gemisch davon, um Vitamin E oder sein Acetat zu erhalten.

## Revendications

1. Procédé pour la fabrication de 3,7-diméthyloct-1-én-3-ol comprenant les étapes suivantes :
a) l'hydrogénation de 6-méthylhept-5-én-2-one en 6-méthylheptan-2-one en présence d'hydrogène et d'un catalyseur contenant du palladium sur un support choisi dans le groupe constitué par le carbone, le carbonate de calcium et l'oxyde d'aluminium ;
b) la réaction de 6-méthylheptan-2-one avec de l'acétylène en 3,7-diméthyloct-1-yn-3-ol en présence d'ammoniac et d'hydroxyde de potassium et en l'absence d'un quelconque solvant organique supplémentaire ;
c) l'hydrogénation de 3,7-diméthyloct-1-yn-3-ol en 3,7-diméthyloct-1-én-3-ol en présence d'hydrogène et d'un catalyseur contenant du palladium sur un support choisi dans le groupe constitué par le carbonate de calcium, l'oxyde d'aluminium, la silice, le verre poreux, le carbone ou le graphite et le sulfate de baryum, à condition que le catalyseur contienne de plus du plomb lorsque le support est du carbonate de calcium ;
dans lequel l'étape a) est mise en oeuvre à une pression dans la plage de 1,1 bar à 15 bar et dans lequel l'étape a) et l'étape c) sont effectuées en l'absence d'un quelconque solvant organique.

2. Procédé selon la revendication 1, dans lequel le catalyseur utilisé dans l'étape a) est le même catalyseur que celui utilisé dans l'étape c).

3. Procédé selon la revendication 2, dans lequel le catalyseur est du Pd sur de l'oxyde d'aluminium.

4. Procédé selon la revendication 1, dans lequel le catalyseur utilisé dans l'étape c) est du palladium sur du carbonate de calcium, dans lequel du plomb est présent.

5. Procédé selon l'une quelconque des revendications précédentes, dans lequel l'étape a) est mise en oeuvre à une température dans la plage de 20 à 100 °C et/ou à une pression dans la plage de 1,5 à 6 bar.

6. Procédé selon l'une quelconque des revendications précédentes, dans lequel la quantité en % en poids de catalyseur utilisé dans l'étape a) est dans la plage de 1:50 à 1:5000, par rapport à la quantité de 6-méthylhept-5-én-2-one en % en poids.

7. Procédé selon l'une quelconque des revendications précédentes, dans lequel le rapport molaire de la 6-méthylheptan-2-one à l'acétylène dans l'étape b) est dans la plage de 1:1 à 1:2 et/ou dans lequel le rapport molaire de la 6-méthylheptan-2-one à l'hydroxyde de potassium dans l'étape b) est dans la plage de 30:1 à 250:1 et/ou dans lequel le rapport molaire de la 6-méthylheptan-2-one à l'ammoniac dans l'étape b) est dans la plage de 1:15 à 1:50.

8. Procédé selon l'une quelconque des revendications précédentes, dans lequel l'étape b) est mise en oeuvre à une température dans la plage de -10 °C à 25 °C et à une pression dans la plage de 5 à 25 bar.

9. Procédé selon l'une quelconque des revendications précédentes, dans lequel l'étape c) est mise en oeuvre à une température dans la plage de 20 °C à 100 °C et/ou à une pression dans la plage de 1,1 à 10 bar.

10. Procédé selon l'une quelconque des revendications précédentes, dans lequel la quantité en % en poids du catalyseur utilisé dans l'étape c) est dans la plage de 1:50 à 1:5000, par rapport à la quantité de 3,7-diméthyloct-1-yn-3-ol en % en poids.

11. Procédé selon l'une quelconque des revendications précédentes, dans lequel le catalyseur utilisé dans l'étape c) comprend une quantité de palladium dans la plage de 1 à 10 % en poids, par rapport au poids total du catalyseur.

12. Procédé selon la revendication 4, dans lequel le catalyseur utilisé dans l'étape c) comprend une quantité de plomb dans la plage de 0 à 9 % en poids, par rapport au poids total du catalyseur.

13. Procédé pour la fabrication de phytol ou d'isophytol comprenant les étapes suivantes
- la préparation de 3,7-diméthyloct-1-én-3-ol selon l'une quelconque des revendications précédentes ;
- la préparation de 6,10-diméthylundéc-5-én-2-one à partir d'un 3,7-diméthyloct-1-én-3-ol ainsi préparé ;
- l'hydrogénation d'une 6,10-diméthylundéc-5-én-2-one ainsi préparée pour obtenir de l'hexahydropseudoionone ;
- la préparation de phytol ou d'isophytol à partir d'une hexahydropseudoionone ainsi préparée.

14. Procédé pour la fabrication de vitamine E ou de son acétate comprenant les étapes suivantes :
- la préparation de 3,7-diméthyloct-1-én-3-ol selon l'une quelconque des revendications 1 à 12 ;
- la préparation de 6,10-diméthylundéc-5-én-2-one à partir d'un 3,7-diméthyloct-1-én-3-ol ainsi préparé ;
- l'hydrogénation d'une 6,10-diméthylundéc-5-én-2-one ainsi préparée pour obtenir de l'hexahydropseudoionone ;
- la préparation de phytol ou d'isophytol à partir d'une hexahydropseudoionone ainsi préparée ;
- la réaction de 2,3,6-triméthylhydroquinone ou de 1-acétate de 2,3,6-triméthylhydroquinone avec du phytol ou de l'isophytol ou un quelconque mélange de ceux-ci pour obtenir de la vitamine E ou son acétate.
